# EUROPÄISCHE PATENTSCHRIFT

(11) **EP 0 316 672 B1**
(45) Veröffentlichungstag und Bekanntmachung des Hinweises auf die Patenterteilung: **09.06.1993**
(21) Anmeldenummer: 88118347.9
(22) Anmeldetag: 04.11.1988
(51) Int. Cl.: C07D 319/06, C07D 319/08

(54) **Verfahren zur Herstellung von Glyoxalmonoacetalen**
Process for the preparation of glyoxal monoacetals
Procédé de préparation de glyoxalmonoacétals

(30) Priorität: 13.11.1987 DE 3738535
(43) Veröffentlichungstag der Anmeldung: 24.05.1989
(73) Patentinhaber: BASF Aktiengesellschaft, 67063 Ludwigshafen (DE)
(72) Erfinder: Merger, Franz, Dr., D-6710 Frankenthal (DE); Frank, Juergen, Dr., D-6830 Schwetzingen (DE)

(56) Entgegenhaltungen:
- EP-A- 0 249 530
- DE-A- 2 514 001
- JOURNAL OF ORGANIC CHEMISTRY, Band 38, Nr. 3, 1973, Seiten 556-560; J.M. KLIEGMAN et al.: "Glyoxal derivatives. V. Reaction of alcohols with glyoxal"

## Beschreibung

Die Erfindung betrifft ein Verfahren Zur Herstellung von Monoacetalen des Glyoxals durch Umsetzung von Glyoxal mit einem substituierten 1,3-Propandiol in Gegenwart von Wasser, einer Säure und einem mit Wasser nicht mischbaren Monoalkanol. Glyoxalmonoacetale sind wertvolle Bausteine für die organische Chemie, da sie gezielte Umsetzungen an nur einer der beiden Carbonylgruppen des Glyoxals erlauben. Folgeprodukte der Glyoxalmonoacetale sind z.B. 3-Methylfumardialdehyd-1-acetale, welche wiederum zur Herstellung pharmakologisch wichtiger Verbindungen, wie z.B. Retinal, verwendet werden können.

Bekanntlich erhält man durch Umsetzung von Glyoxal mit aliphatischen Mono- oder Dialkoholen unter Abtrennung von Wasser die entsprechenden Diacetale des Glyoxals. Diese Umsetzung verlauft in der Regel nicht mit hoher Selektivität und man beobachtet eine Reihe von Nebenreaktionen. Insbesondere findet man Verbindungen mit höheren Molekulargewichten, die durch Vereinigung mehrerer Glyoxaleinheiten und mehrerer Alkoholmoleküle gebildet werden. So ist in J. Am. Chem. Soc. 77, 1285 (1955) beschrieben, daß bei der Acetalisierung von Glyoxallösung mit n-Butanol neben dem erwarteten 1,1,2,2-Tetrabutoxyethan auch 2,3,5,6-Tetrabutoxy-1,4-dioxan gebildet wird. Aus J. Org. Chem. 37, 1276 (1972) und 38, 556 (1973) sowie insbesondere aus Bull. Soc. Chim. France (5), 822 (1986) geht hervor, daß die Verhältnisse bei der Umsetzung von Glyoxal mit Alkoholen sogar noch wesentlich komplizierter sind und eine Vielzahl verschiedener Kondensationsprodukte entsteht.

Bekannte Umsetzungen von Glyoxal mit Diolen führen zu Diacetalen und unterliegen ähnlich komplizierten Verhältnissen wie sie bei Umsetzungen des Glyoxals mit Monoalkoholen beobachtet werden. Bereits in Monatsh. Chem. 16, S. 14ff (1895) ist die zu einem Glyoxaldiacetal führende Umsetzung zwischen Glyoxal und Glykol beschrieben. Nach J. Chem. Soc. (London) 86, 1932 wird diesem Diacetal die Konstitution eines 1,4,5,8-Tetraoxadecalins zugeschrieben. In Receuil Trav. Chim. Pays-Bas 50, 909 (1931) ist beschrieben, daß eine beabsichtigte unabhängige Synthese des Tetraoxadecalins zum Teil auch zu einem Isomeren fuhrt. Dessen Identität als Di-(1,3-dioxolan-2-yl) ist nach den Angaben in Acta Chem. Scand. 4, 965 (1950) durch Kristallstrukturanalyse bewiesen. Nach erneuten Untersuchungen wurde in Chem. Ber. 87, 1343 (1954) festgestellt, daß durch azeotrope Acetalisierung ausgehend von Glyoxallösung immer Isomerengemische erhalten werden.

Fur die Herstellung von Monoacetalen des Glyoxals hat man auf der Grundlage dieses Wissens zu anderen Methoden greifen müssen. So hat man Monoacetale des Glyoxals durch eine Spaltung von Acetalen des Crotonaldehyds mit Ozon hergestellt (DE-OS 25 14 001, DE-OS 25 13 999 und Synth. Commun. 1976, 141.). Auf diese Weise wurden Monoacetale des Glyoxals mit Mono- und Dialkoholen erhalten. Insbesondere konnte so das Monoacetal des Glyoxals mit 2,2-Dimethyl-1,3-propandiol (Neopentylglykol) gewonnen werden. Nach den Angaben in Synthesis 1981, 129 erhält man das Mono-diethylacetal des Glyoxals auch durch Ozonspaltung des Diethylacetals des Acroleins. In Helv. Chim. Acta 18, 514 (1935) und Ann. Chim. Vol. 2, 87 (1977) ist beschrieben, daß man Glyoxalmonoacetale durch Spaltung von Acetalen des gegebenenfalls substituierten Glycerinaldehyds mit Blei(+IV)-Salzen herstellen kann. Die dafür benötigten Acetale des substituierten Glycerinaldehyds sind aus Acetalen des Acroleins bzw. des Crotonaldehyds durch Behandlung mit Kaliumpermanganat zugänglich (s.a. J. Am. Chem. Soc. 51, 3115 (1929)).

Diese Synthesen haben erhebliche wirtschaftliche und verfahrenstechnische Nachteile. So ist die Herstellung von Ozon aufwendig. Außerdem muß bei dessen Verwendung im allgemeinen in hoher Verdünnung gearbeitet werden, um die Ansammlung explosionsgefährlichen Materials zu vermeiden. Die Herstellung von Glyoxalmonoacetalen mit Blei(+IV)-Salzen ist durch die Toxizität der Bleiverbindungen und das kostspielige Kaliumpermanganat belastet. Darüber hinaus werden die Glyoxalmonoacetale erst nach einer vierstufigen Reaktionsfolge in unbefriedigender Ausbeute erhalten.

Zum Stande der Technik nach Art. 54(3) EPÜ gehört ein in der der EP-A-249 530 beschriebenes Verfahren zur Herstellung von Glyoxalmonoacetalen, bei dem man Glyoxal in Anwesenheit eines sauren Katalysators mit einem Überschuß eines Alkohols umsetzt und dann die Reaktion abbricht, sobald die Konzentration an gewünschtem Monoacetal im Reaktionsmedium zugunsten des Diacetals nachläßt, wobei die Reaktion durch Analyse von regelmäßig aus dem Reaktionsmedium entnommenen Proben überwacht wird. Zur Durchführung dieser Reaktion werden als Lösungsmittel namentlich Hexan, Cyclohexan, Benzol, Toluol, Chloroform und Dichlormethan empfohlen.

Es wurde nun gefunden, daß man Monoacetale des Glyoxals der allgemeinen Formel 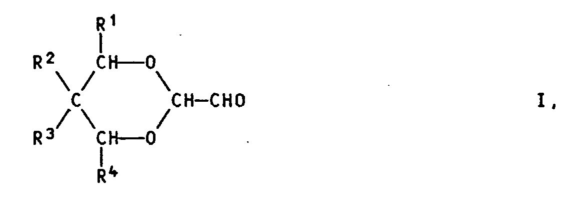
in der R¹, R², R³ und R⁴ aliphatische Reste mit 1 bis 6 C-Atomen oder aromatische Reste bedeuten, bis zu drei der Reste R¹, R², R³ und R⁴ auch für Wasserstoffatome stehen können und die Reste R² und R³ oder R¹ und R² auch jeweils gemeinsam Glieder eines aliphatischen 4- bis 7-gliedrigen Ringes, der noch ein Heteroatom enthalten kann, bedeuten können, auf technisch besonders vorteilhafte Weise dadurch herstellen kann, daß man Glyoxal mit einem 1,3-Propandiol der Formel 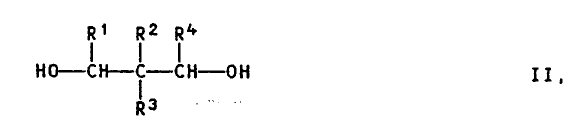
in der die Reste R¹ bis R⁴ die obengenannte Bedeutung haben, in Gegenwart von Wasser, einer sag und einem Monoalkanol, welches mit dem wäßrigen Ausgangsgemisch zwei Phasen bildet, bei Temperaturen bis 150°C umsetzt, wobei man pro Mol Glyoxal 0,1 bis 5 Mol des 1,3-Propandiols einsetzt.

Die 1,3-Propandiole der Formel II enthalten als aliphatische Reste mit 1 bis 6 C-Atomen z.B. geradkettige, verzweigte oder cycloaliphatische Reste, wie Alkylgruppen mit 1 bis 6, vorzugsweise 1 bis 4 C-Atomen oder cycloaliphatische Reste mit 4 bis 6 C-Atomen. Die Reste R² und R³ oder R¹ und R² können auch jeweils gemeinsam Glieder eines aliphatischen 4- bis 7-gliedrigen, vorzugsweise 5- bis 6-gliedrigen Ringes sein, der ein Heteroatom, wie ein Sauerstoff- oder Stickstoffatom enthalten kann. Beispielsweise seien die folgenden aliphatischen Reste genannt: Methyl, Ethyl, Propyl, Isopropyl, Butyl und Cyclohexyl. Als aromatische Reste kommen z.B. Phenylreste in Betracht. Aliphatische 4- bis 7-gliedrige Ringe, die noch ein Heteroatom enthalten können, sind z.B. Cyclopentan, Cyclohexan oder Tetrahydropyran. Im einzelnen seien die folgenden 1,3-Propandiole genannt: 2,2-Dimethylpropandiol-(1,3), 2-Ethyl-2-methylpropandiol-(1,3), 2-Butyl-2-ethylpropandiol-(1,3), 1-Isopropyl-2,2-dimethyl-propandiol-(1,3) und 1-Propyl-2-ethylpropandiol-(1,3). 1,1-Dihydroxymethyl-cyclopentan, 1,1-Dihydroximethyl-cyclohexan und 3,3-Dihydroxymethyl-tetrahydropyran.

Das Glyoxal wird vorzugsweise als wäßrige Lösung eingesetzt, wobei man zweckmäßigerweise die üblichen technischen wäßrigen Lösungen mit einem Glyoxalgehalt von 20 bis 60, vorzugsweise 30 bis 50 Gew.% verwendet. Pro Mol Glyoxal werden 0.1 bis 5 Mol, vorzugsweise 0,5 bis 2 Mol, insbesondere 0,6 bis 0,9 Mol des 1 ,3-Propandiols der Formel II eingesetzt.

Man setzt das Glyoxal mit dem 1,3-Propandiol in Gegenwart von katalytischen Mengen Säure um. Als Säuren kommen z.B. Schwefelsäure, Methansulfonsäure, p-Toluolsulfonsäure, saure Ionenaustauscherharze, Pyridinium-p-toluolsulfonat, Trichloressigsäure und Oxalsäure in Betracht. Im allgemeinen verwendet man die Säuren in Mengen von 0,01 Mol bis 0,25 Mol pro Mol Glyoxal. Bei Festlegung der zu verwendenden Säuremenge empfiehlt es sich, die optimalen Mengen in einem Vorversuch zu ermitteln. Eine große Säuremenge führt zwar zu einer gewünschten hohen Reaktionsgeschwindigkeit, sie kann aber auch die Oligomerisierung des Glyoxals begünstigen und zur Bildung von Nebenreaktionen Anlaß geben.

Bei dem im Reaktionsmedium enthaltenen, mit Wasser nicht mischbaren Monoalkanol kann es sich z.B. um Butanole, Pentanole, Hexanole, Ethylhexanol, Nonanole, Decanole und Undecanole, handeln.

Ausser dem genannten Monoalkanol kann das Reaktionsmedium weitere Lösungsmittel enthalten, welche mit dem wäßrigen Ausgangsgemisch zwei Phasen bilden. Das sind Losungsmittel, die im Temperaturbereich von 20 bis 150°C nicht mit Wasser mischbar sind. Lösungsmittel dieser Art sind z.B.aliphatische, cycloaliphatische, oder aromatische Kohlenwasserstoffe, die z.B. Halogenatome, Hydroxygruppen und/oder Alkoxygruppen enthalten können, und 2 bis 15 Kohlenstoffatome aufweisen. Das sind z.B. aliphatische und cycloaliphatische Kohenwasserstoffe, wie n-Hexan, iso-Hexan, isomere Heptane, Octane, Cyclohexan, Halogenalkane, wie 1,2-Dichlorethan, 1,2-Dichlorpropan, Trichlormethan, Tetrachlormethan, 1,1,1- und 1,1,2-Trichlorethan, 1,1,2-Trichlorethen, oder gegebenenfalls substituierte aromatische Kohlenwasserstoffe, wie Benzol, Toluol, Xylol, Ethylbenzol, Isopropylbenzol, Durol, Tetralin, Chlorbenzol, 1,2-Dichlorbenzol oder Anisol. Die Losungsmittel, die auch im Gemisch eingesetzt werden können, werden, z.B. in Mengen von 100 bis 2000 ml, vorzugsweise 150 bis 1000 ml, insbesondere 200 bis 500 ml pro Mol eingesetzten Diols zugegeben. Besonders vorteilhafte Ergebnisse werden erhalten, wenn man als Lösungsmittel Gemische aus Alkanolen, wie Butanol, Pentanol oder Hexanol einerseits und aromatischen Kohlenwasserstoffen, wie Benzol, Toluol oder Xylol andererseits verwendet. So erhalt man z.B. beim Einsatz eines Lösungsmittelgemisches aus 1 Gew. -Teil der genannten Alkanole und 2 bis 6 Gew.-Teilen der genannten aromatischen Kohlenwasserstoffe überraschenderweise sogar in den Fällen hohe Ausbeuten, in denen man die Propandiole im Unterschuß (d.h. weniger als die molare Menge, bezogen auf eingesetztes Glyoxal) einsetzt.

Man kann das Verfahren bei Normaldruck, vermindertem oder erhöhtem Druck durchfuhren. Bei Umsetzungen unter erhöhtem Druck, vorzugsweise bei Drücken bis 5 bar, kann man die Reaktionszeit bei erhöhter Selektivität vorteilhaft erniedrigen. Damit ist die Möglichkeit gegeben, durch Wahl des Lösungsmittels und der Reaktionstemperatur, Nebenreaktionen (z.B. Umlagerungen) zurückzudrangen. Umlagerungen treten vor allem bei höheren Temperaturen ein. Man arbeitet deshalb in einem Temperaturbereich von z.B. 20 bis 150°C, vorzugsweise 50 bis 140°C und insbesondere bei 80 bis 130°C.

Das neue Verfahren eignet sich gleichermaßen für die diskontinuierliche wie auch die kontinuierliche Arbeitsweise. Dabei richten sich die erforderlichen Verweilzeiten sowohl nach der Wahl des Lösungsmittels und der Reaktionstemperatur als auch nach der Art und Güte der Durchmischung der Reaktionsmasse. Als einsetzbare Reaktoren stehen z.B. kontinuierlich oder diskontinuierlich betriebene Rührkessel, Mixer-Settler-Batterien oder Kolonnen, mit und ohne Einbauten, zur Auswahl. Die Verweilzeit kann folglich von Fall zu Fall sehr unterschiedlich sein und 30 Minuten bis 40 Stunden betragen; sie liegt zweckmäßig im Bereich zwischen 45 Minuten und 12 Stunden.

Sowohl bei der diskontinuierlichen als auch bei kontinuierlicher Arbeitsweise ist zwar der erreichbare Umsatz durch die Einstellung des Acetalisierungsgleichgewichtes begrenzt, doch kann man nach der Isolierung des Acetals der Formel I durch Phasentrennung, Neutralisation des Katalysators und fraktionierende Destillation der organischen Phase den Destillationsrückstand mit der wäßrigen Phase und gegebenenfalls Lösungsmittel wieder vereinigen und erneut eine Gleichgewichtsstellung erreichen. Durch diese Rückführung wird es möglich, hohe Gesamtausbeuten zu erreichen und damit das Verfahren besonders wirtschaftlich zu gestalten.

Bei dem erfindungsgemäßen Verfahren, besonders in der bevorzugten Ausführungsform, wird überraschenderweise die Bildung von Glyoxaldiacetalen fast völlig unterdruckt und damit die Herstellung der Glyoxalmonoacetale mit hoher Selektivität ermöglicht.

Die nach dem erfindungsgemäßen Verfahren erhältlichen neuen Glyoxalmonoacetale sind wertvolle Zwischenprodukte, z.B. für die Herstellung von Carotinoiden.

### Beispiel 1

In einem Autoklaven wurden 6525 g (45 Mol) einer 40 %igen Lösung von Glyoxal in Wasser, 4680 g (45 Mol) 2,2-Dimethylpropandiol-(1,3), 3330 g (45 Mol) n-Butanol und 18,0 l Toluol bei 80°C vorgelegt. Dann wurden 450 g 50 %ige Schwefelsäure zugegeben, der Autoklav wurde verschlossen und auf 120°C aufgeheizt. Man rührte eine Stunde bei 120°C und erhielt nach Abkühlen 25,4 kg organische Phase, die der gaschromatographischen Analyse zufolge 4710 g 2-Formyl-5,5-dimethyl-1,3-dioxan enthielt.

### Beispiel 2

Ein Gemisch aus 145 g (1 Mol) einer 40 %igen Lösung von Glyoxal in Wasser, 144 g (1 Mol) 1,1-Dimethylolcyclohexan, 5 g konz. Schwefelsäure, 400 ml Xylol und 74 g n-Butanol wurde 18 Stunden in der Siedehitze gerührt. Die organische Phase wog 587 g und enthielt nach gaschromatographischer Analyse 117 g 3-Formyl-2,4-dioxaspiro(5,5)-undecan (Glyoxal-mono-1,1-dimethylolcyclohexan-acetal). Die Substanz wurde durch Rückstandsdestillation isoliert. Der Siedepunkt betrug 120 bis 125°C/1 mbar.

### Beispiel 3

In einem Glasautoklaven wurde eine Mischung aus 78 g (0,75 Mol) 2,2-Dimethylpropandiol-(1,3), 108,8 g (0,75 Mol) 40 %iger wäßriger Glyoxallösung, 55,5 g (0,75 Mol) n-Butanol und 240 g Toluol bei 120°C vorgelegt und 10 g 50 %ige Schwefelsaure zugepumpt. Man rührte 2 h bei 120°C, kühlte ab und trennte die Phasen. Die organische Phase wog 312 g und enthielt der gaschromatographischen Analyse zufolge 63,6 g 2-Formyl-5,5-dimethyl-1,3-dioxan, entsprechend 59 % Ausbeute.

### Beispiel 4

In einem Glasautoklaven wurde eine Mischung aus 58,5 g (0,56 Mol) 2,2-Dimethylpropandiol-(1,3), 108,8 g (0,75 Mol) 40 %iger wäßriger Glyoxallösung, 55,5 g (0,75 Mol) n-Butanol und 240 g Toluol bei 120°C vorgelegt und 10 g 50 %ige Schwefelsäure zugepumpt. Man rührte 2 h bei 120°C, kühlte ab und trennte die Phasen. Die organische Phase wog 398 g und enthielt der gaschromatographischen Analyse zufolge 59,3 g 2-formyl-5,5-dimethyl-1,3-dioxan, entsprechend 74 % Ausbeute.

### Beispiel 5

In einem Glasautoklaven wurde eine Mischung aus 81,8 g (0,56 Mol) 2,2,4-Trimethylpentandiol-(1,3), 108,8 g (0,75 Mol) 40 %iger wäßriger Glyoxallösung, 55,5 g (0,75 Mol) n-Butanol und 240 g Toluol bei 120°C vorgelegt und 10 g 50 %ige Schwefelsäure zugepumpt. Man rührte 2 h bei 120°C, kühlte ab und trennte die Phasen. Die organische Phase wog 421 g und enthielt der gaschromatographischen Analyse zufolge 84 g 2-Formyl-4-isopropyl-5,5-dimethyl-1,3-dioxan, entsprechend 81 % Ausbeute.

## Patentansprüche

1. Verfahren zur Herstellung von Glyoxalmonoacetalen der allgemeinen Formel 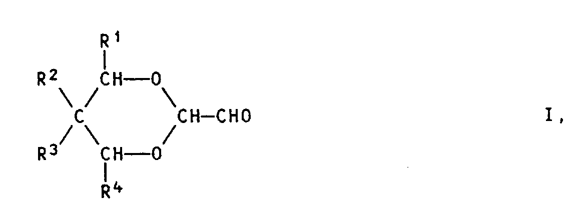 in der R¹, R², R³ und R⁴ aliphatische Reste mit 1 bis 6 C-Atomen oder aromatische Reste bedeuten, wobei bis zu drei der Reste R¹ , R², R³ und R⁴ auch für Wasserstoffatome stehen können und die Reste R² und R³ oder R¹ und R² auch jeweils gemeinsam Glieder eines aliphatischen 4- bis 7-gliedrigen Ringes, der noch ein Heteroatom enthalten kann, bedeuten können, dadurch gekennzeichnet, daß man Glyoxal mit einem 1,3-Propandiol der Formel 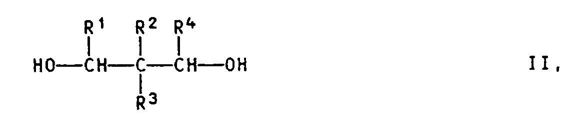 in der die Reste R¹ bis R⁴ die obengenannte Bedeutung haben, in Gegenwart von Wasser, einer Saure und einem Monoalkanol, welcher mit dem wäßrigen Ausgangsgemisch zwei Phasen bildet, bei Temperaturen bis 150°C umsetzt, wobei man pro Mol Glyoxal 0,1 bis 5 Mol des 1,3-Propandiols einsetzt.

2. Verfahren nach Anspruch 1, dadurch gekennzeichnet, daß man zusätzlich zum Monoalkanol weitere Lösungsmittel aus der Gruppe aliphatischer, cycloaliphatischer oder aromatischer Kohlenwasserstoffe, die Halogenatome, Alkoxygruppen und/oder Hydroxygruppen enthalten können und 2 bis 15 Kohlenstoffatome aufweisen, einsetzt, welche mit dem wäßrigen Ausgangsgemisch zwei Phasen bilden.

3. Verfahren nach Anspruch 2, dadurch gekennzeichnet, daß man zusätzlich zum Monoalkanol einen aromatischen Kohlenwasserstoff einsetzt.

4. Verfahren nach Anspruch 3, dadurch gekennzeichnet, daß man ein Lösungsmittelgemisch aus Butanol, Pentanol oder Hexanol einerseits und Benzol, Toluol oder Xylol andererseits verwendet.

5. Verfahren nach Anspruch 4, dadurch gekennzeichnet, daß man ein Losungsmittelgemisch verwendet, das auf 1 Gew.-Teil Butanol, Pentanol oder Hexanol 2 bis 6 Gew.-Teile Benzol, Toluol oder Xylol enthält.

6. Verfahren nach Anspruch 1, dadurch gekennzeichnet, daß man nach Abtrennung des Acetals der Formel I aus dem Reaktionsgemisch die dabei anfallenden wäßrigen und organischen Anteile vereinigt und das so erhaltene Gemisch, gegebenenfalls nach Zugabe von frischem Losungsmittel, zur Bildung weiterer Anteile des Acetals auf Temperaturen bis 150°C erhitzt.

## Claims

1. A process for preparing glyoxal monoacetals of the formula 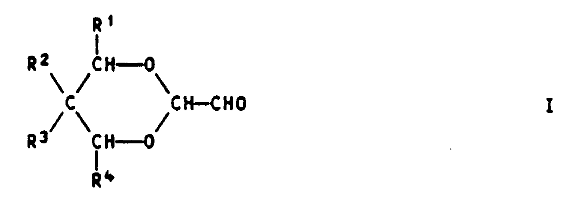 where R', R², R³ and R⁴ are each aliphatic radicals of 1 to 6 carbons or aromatic radicals, it also being possible for up to three of R¹, R², R³ and R⁴ to be hydrogen, and for R² and R³ or R¹ and R⁴ in each case together to be members of an aliphatic 4- to 7-membered ring which may also contain a hetero atom, which comprises reacting glyoxal with a 1,3-propanediol of the formula 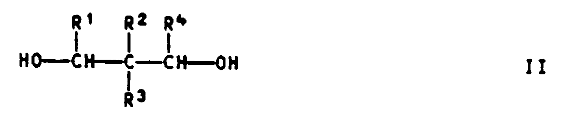 where R¹ to R⁴ have the abovementioned meanings, in the presence of water, of an acid and of a monoalkanol which forms two phases with the initial agueous mixture, at up to 150°C, employing from 0.1 to 5 mol of the 1,3-propanediol per mol of glyoxal.

2. A process as claimed in claim 1, in which are employed, in addition to the monoalkanol, further solvents from the group of aliphatic, cycloaliphatic or aromatic hydrocarbons which can contain halogen atoms, alkoxy groups and/or hydroxyl groups and which have from 2 to 15 carbons, which form two phases with the initial aqueous mixture.

3. A process as claimed in claim 2, wherein an aromatic hydrocarbon is employed in addition to the monoalkanol.

4. A process as claimed in claim 3, wherein a solvent mixture composed of butanol, pentanol or hexanol on the one hand and benzene, toluene or xylene on the other hand is used.

5. A process as claimed in claim 4, wherein a solvent mixture which contains from 2 to 6 parts by weight of benzene, toluene or xylene for 1 part by weight of butanol, pentanol or hexanol is used.

6. A process as claimed in claim 1, wherein, after removal of the acetal of the formula I from the reaction mixture, the aqueous and organic portions resulting from this are combined, and the mixture obtained in this way is, where appropriate after addition of fresh solvent, heated at up to 150°C to form further portions of the acetal.

## Revendications

1. Procédé de préparation de glyoxalmonoacétals de formule générale 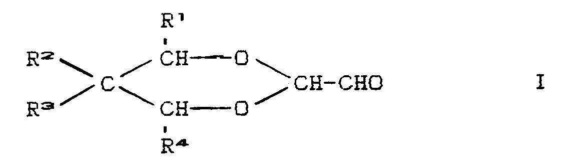 dans laquelle R¹, R², R³ et R⁴ représentent des restes aliphatiques à 1-6 atomes de carbone ou des restes aromatiques, jusqu'à trois des restes R¹, R², R³ et R⁴ pouvant être également mis pour des atomes d'hydrogène et les restes R² et R³ ou R¹ et R² pouvant aussi représenter ensemble, chaque fois, des maillons d'un noyau aliphatique à 4-7 termes cycliques, qui peut encore contenir un hétéroatome, caractérisé en ce qu'on fait réagir du glyoxal avec un 1,3-propanediol de formule 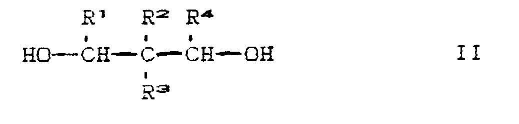 dans laquelle les restes R¹ à R⁴ ont les significations sus-indiquées, en présence d'eau, d'un acide et d'un monoalcanol qui forme deux phases avec le mélange aqueux de départ, à des températures allant jusqu'à 150°C, en utilisant de 0,1 à 5 moles du 1,3-propanediol par mole de glyoxal.

2. Procédé selon la revendication 1, caractérisé en ce qu'on utilise, en plus du monoalcanol, d'autres solvants du groupe des hydrocarbures aliphatiques, cycloaliphatiques ou aromatiques, qui peuvent contenir des atomes d'halogène, des groupements alcoxy et/ou des groupements hydroxy et qui comportent de 2 à 15 atomes de carbone, solvants qui forment deux phases avec le mélange aqueux de départ.

3. Procédé selon la revendication 1, caractérisé en ce qu'on utilise, en plus du monoalcanol, un hydrocarbure aromatique.

4. Procédé selon la revendication 3, caractérisé en ce qu'on utilise un mélange de solvants composé de butanol, de pentanol ou d'hexanol d'une part et de benzène, de toluène ou de xylène d'autre part.

5. Procédé selon la revendication 4, caractérisé en ce qu'on utilise un mélange de solvants qui contient de 2 à 6 parties en poids de benzène, de toluène ou de xylène pour 1 partie en poids de butanol, de pentanol ou d'hexanol.

6. Procédé selon la revendication 1, caractérisé an ce qu'après avoir séparé du mélange réactionnel l'acétal de formule I, on réunit les fractions aqueuse et organique qui sont alors produites et on chauffe à des températures allant jusqu'à 150°C le mélange ainsi obtenu, éventuellement après addition de solvant frais, pour la formation de parts supplémentaires de l'acétal.
